Europäisches Patentamt

European Patent Office

Office européen des brevets

(11) Veröffentlichungsnummer: **0 460 673 A1**

## (12) EUROPÄISCHE PATENTANMELDUNG

(21) Anmeldenummer: 91109302.9

(22) Anmeldetag: 06.06.91

(51) Int. Cl.5: **C12N 15/55**, C12N 15/54, C12N 9/22, C12N 9/10

(30) Priorität: 08.06.90 DE 4018441

(43) Veröffentlichungstag der Anmeldung:
11.12.91 Patentblatt 91/50

(84) Benannte Vertragsstaaten:
AT BE CH DE DK ES FR GB GR IT LI LU NL SE

(71) Anmelder: **BOEHRINGER MANNHEIM GMBH
Sandhofer Strasse 112-132
W-6800 Mannheim-Waldhof(DE)**

(72) Erfinder: **Götz, Friedrich, Prof. Dr.
Beim Herbstenhof 31
W-7400 Tübingen(DE)**
Erfinder: **Seeber, Stefan, Dr.
Listseeweg 7
W-8000 München 70(DE)**

(74) Vertreter: **Huber, Bernhard, Dipl.-Chem. et al
Möhlstrasse 22 Postfach 860 820
W-8000 München 86(DE)**

(54) Rekombinantes Restriktionsenzym Sau3AI.

(57) Die Erfindung betrifft eine DNA, die
(1) mindestens einen der beiden für ein Protein mit einer Sau3AI-Methylase-Aktivität bzw. für ein Protein mit einer Sau3AI-Endonuklease-Aktivität kodierenden Bereiche der in SEQ ID NO:1 dargestellten Nukleotidsequenz,
(2) eine der DNA aus (1) im Rahmen der Degeneration des genetischen Codes entsprechende Sequenz oder
(3) eine Sequenz, die unter stringenten Hybridisierungsbedingungen mit einer DNA aus (1) oder (2) hybridisiert, enthält.
Weiterhin wird ein Vektor offenbart, der eine erfindungsgemäße DNA, insbesondere unter Kontrolle eines regulierbaren dichten Promotors enthält, sowie Mikroorganismen, die mit einem oder mehreren erfindungsgemäßen Vektoren transformiert sind, und die Gewinnung von rekombinanter Sau3AI-Endonuklease aus den transformierten Mikroorganismen.

EP 0 460 673 A1

EP 0 460 673 A1

Restriktions-Modifikations (R-M)-Systeme der Klassen I, II und III in Bakterien stellen Verteidigungsmechanismen gegen eindringende DNA-Moleküle dar. Sie bestehen aus zwei enzymatischen Aktivitäten:

1. einer Restriktions-Endonuklease, welche nicht modifizierte und somit ungeschützte DNA an einer spezifischen Erkennungsstelle erkennt und spaltet und

2. einer DNA-Modifikations-Methyltransferase, welche die zelluläre DNA durch spezifische Methylierung von mindestens einer speziellen Base in der gleichen Sequenz vor der Spaltung durch die Restriktions-Endonuklease schützt.

Auf diese Weise ermöglichen die verschiedenen R-M-Systeme dem Bakterium eine Resistenz gegenüber einer Phagen-Infektion. Diese Phagen-Resistenz war die Basis für die Entwicklung des internationalen Phagen-Klassifikations-Systems für Staphylococcus aureus (Williams und Rippon, J. Hyg. 50 (1952), 320-353), um verschiedene Stämme von S. aureus zu charakterisieren. Gemäß ihrer Phagen-Klassifikation können S. aureus Stämme in vier Gruppen eingeteilt werden. Die Unterschiede zwischen den einzelnen Gruppen sind hauptsächlich durch die R-M-Aktivität bedingt. Sussenbach et al. (Nucleic Acids Res. 3 (1976), 3193-3202) konnten das Klasse II-Restriktionsenzym Sau3AI (oder R• Sau3AI) aus dem Gruppe II-Stamm S. aureus 3AI isolieren. Es erkennt die palindrome Sequenz 5'- GATC-3' und spaltet vor dem G-Rest auf jedem DNA-Strang. Die Sau3AI-Methyltransferase (M• Sau3AI) modifiziert die C-Reste beider Stränge an Position $^5$C, so daß eine Sequenz 5,- GAT$^{5\text{-Me}}$C-3' entsteht, die von dem Restriktionsenzym nicht gespalten werden kann.

Das Restriktionsenzym Sau3AI ist seit vielen Jahren bekannt. Es wird aus Staphylococcus aureus 3A gewonnen und hat sich als eines der bedeutsamsten Restriktionsenzyme für die rekombinante DNA-Technologie (z.B. in Verbindung mit BamHI (5'-G/GATCC-3') zur Herstellung von Genbanken) erwiesen. Die bisher noch nicht erhältliche Methylase M• Sau3AI könnte ebenso von großem Interesse zur Methylierung von DNA sein.

Die Isolierung von Sau3AI bereitet insofern Probleme und hohe Kosten, da der Wirtsorganismus S. aureus pathogen und schlecht aufschließbar ist, wodurch eine Gewinnung des Enzyms erheblich erschwert wird. Weiterhin enthält S. aureus auch störende Nebenaktivitäten, welche die Reinigung und Gewinnung des Enzyms zusätzlich behindern.

Somit existiert ein großes Bedürfnis, das Restriktions-Modifikations-System Sau3AI in einen geeigneteren Organismus zu klonieren und dort zu exprimieren, so daß eine bessere Anreicherung des Restriktionsenzyms möglich ist. Der am besten hierfür geeignete Organismus wäre das gram-negative Bakterium E. coli.

Zur Klonierung von Restriktions-Modifikations-Systemen aus verschiedenen Organismen in E. coli gibt es mehrere bekannte Verfahren, die bei Wilson (Gene 74 (1988), 281-288) beschrieben sind. Zu diesen Verfahren gehören z.B. die Subklonierung von natürlichen Plasmiden, auf denen sich die Gene eines R-M-Systems befinden oder eine Selektion aufgrund von Phagenrestriktion. Üblicherweise wird jedoch eine Selektions-Strategie verwendet, bei der man eine Genbank von genomischen DNA-Fragmenten des Ausgangsorganismus in E. coli anlegt und eine in vitro Selektion auf Methylase-positive Klone durchführt. Auf diese Weise konnten bereits über 50 Modifikationsgene isoliert werden, davon viele zusammen mit dem jeweils korrespondierenden Restriktionsgen, das sich im allgemeinen direkt benachbart zum Methylasegen befindet. Mit diesem bekannten Verfahren ist es jedoch nicht möglich, das Sau3AI-R-M-System zu klonieren.

Aufgabe der vorliegenden Erfindung war es, diese Schwierigkeiten zu überwinden und eine Möglichkeit zur Klonierung und Expression des Restriktionsenzyms Sau3AI zu finden.

Die erfindungsgemäße Aufgabe wird durch die Bereitstellung einer DNA gelöst, welche

(1) mindestens einen der beiden für ein Protein mit einer Sau3AI-Methylase-Aktivität bzw. für ein Protein mit einer Sau3AI-Endonuklease-Aktivität kodierenden Bereiche der Nukleotidsequenz SEQ ID NO:1,

(2) eine der DNA aus (1) im Rahmen der Degeneration des genetischen Codes entsprechenden Sequenz oder

(3) eine Sequenz, die unter stringenten Hybridisierungsbedingungen mit einer DNA aus (1) oder (2) hybridisiert,

enthält.

Überraschenderweise gelingt die Klonierung der Gene des Sau3AI-Restriktions-Modifikations-Systems, wenn ein Wirtsorganismus verwendet wird, der mit S. aureus 3AI nahe verwandt ist, aber das Sau3AI-Restriktions-Modifikations-System nicht besitzt. Vorzugsweise verwendet man als mit S. aureus nahe verwandten Organismus das nicht pathogene Bakterium Staphylococcus carnosus, DSM 5875, jedoch ohne Plasmid (Schleifer und Fischer, Int. J. Syst. Bact. 32 (1982), 153-156; Götz et al., Plasmid 9 (1983)). Weitere geeignete Stämme zur Klonierung des Sau3AI-R-M-Systems sind z.B. Staphylococcus xylosus C2A und von S. aureus 3AI verschiedene Staphylococcus aureus Stämme, welche das Sau3AI-R-M-System nicht

2

enthalten. Auf diese Weise ist es möglich, eine erfindungsgemäße DNA zu erhalten, welche beide Gene des Sau3AI-R-M-Systems vollständig enthält.

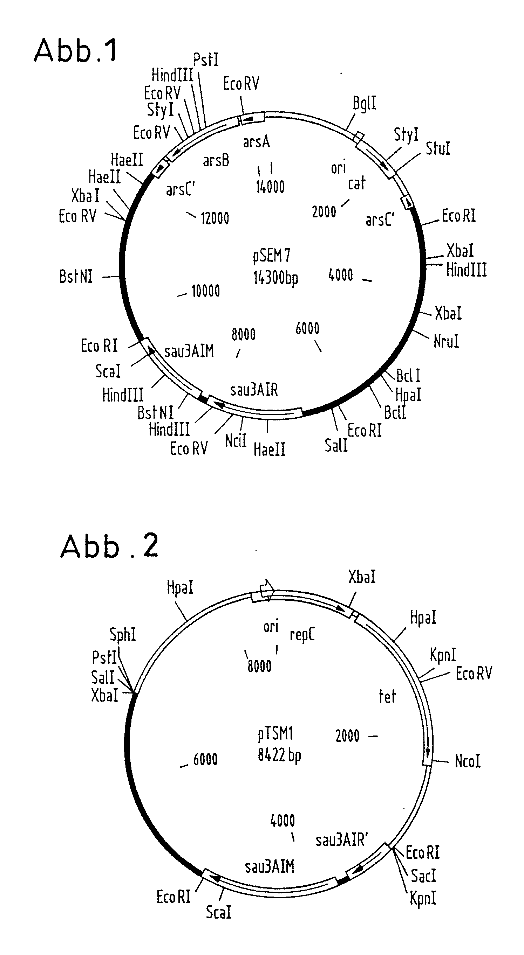

Ein rekombinanter Vektor, welcher eine erfindungsgemäße DNA mit beiden Genen des Sau3AI-R-M-Systems enthält, ist das Plasmid pSEM7 (Abb. 1). Staphylococcus carnosus, transformiert mit dem Plasmid pSEM7, wurde bei DSM unter der Nummer 5875 hinterlegt. Fragmente mit den unvollständigen Genen des Sau3AI R-M-Systems wurden in den E. coli Vektor pUC19 kloniert. Ihre Nukleotidsequenz wurde nach der Didesoxy-Methode von Sanger bestimmt.

Die Nukleotidsequenz des Sau3AI-R-M-Systems zeigt zwei große offene Leserahmen, welche in dieselbe Richtung transkribiert werden (SEQ ID NO:1). Aufgrund der Kartierung und Proteinsequenz-Daten können die Gene für die Sau3AI-Methylase und für die Sau3AI-Restriktionsendonuklease eindeutig lokalisiert werden. R* Sau3AI wird durch einen offenen Leserahmen kodiert, der sich von Base 448 bis Base 1914 erstreckt. M* Sau3AI wird durch den offenen Leserahmen kodiert, der bei Base 2017 beginnt und bei Base 3252 endet.

Das Sau3AIR-Gen beginnt mit dem Startcodon TTG. Die Identität und der Translationsstart des Sau3AI-R-Leserahmens wurden durch Sequenzierung der ersten 23 N-terminalen Aminosäuren des gereinigten Enzyms bestätigt. Demnach besteht R* Sau3AI aus 489 Aminosäuren mit einem berechneten Molekulargewicht von 56,5 kD. Dies stimmt mit dem Wanderungsverhalten von gereinigtem R* Sau3AI bei der SDS-PAGE überein.

Der Leserahmen des Sau3AI M-Gens kodiert für ein Protein mit 412 Aminosäuren mit einem berechneten Molekulargewicht von 47,3 kD. Beide Gene des R-M-Systems besitzen einen G + C-Gehalt von 31 %.

Auf diese Weise konnte die Sau3AI-Methylase-Aktivität alleine auf einem 3,7 kb großen EcoRV-Fragment subkloniert werden. Das resultierende Plasmid pTSM1 ist 8,4 kb groß und trägt neben dem aktiven Methylasegen noch ein kurzes inaktives Fragment des Endonukleasegens (siehe Abb. 2).

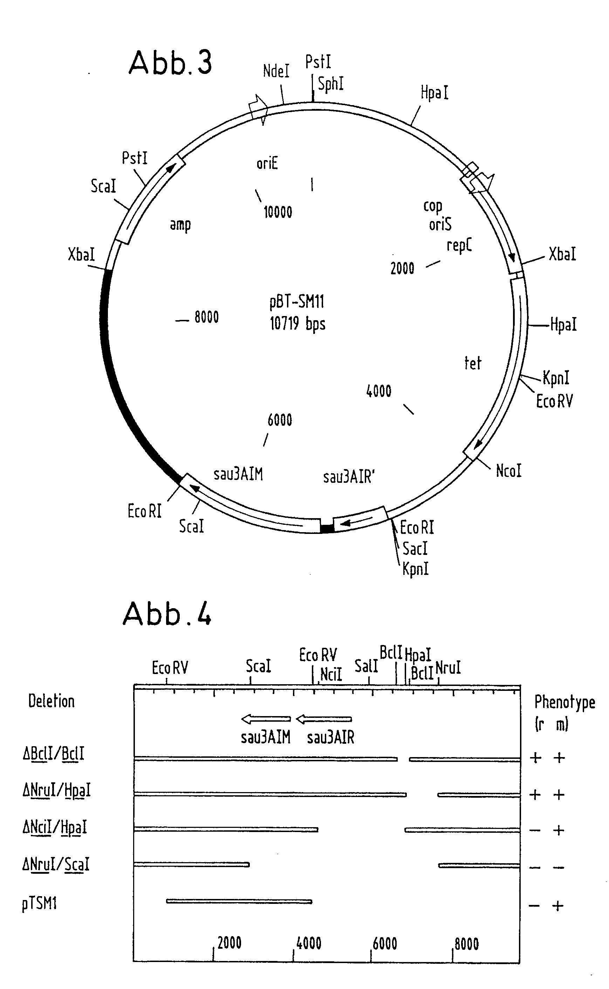

Durch Insertion eines 2,3 kb Fragmentes (EcoRI, PvuII, stumpfe Enden durch Behandlung mit Klenow Enzym) des E. coli Vektors pBR322 in die SalI-Schnittstelle (ebenso stumpfe Enden durch Klenow-Behandlung) von pTSM1 konnte der S. carnosus/E. coli Shuttle Vektor pBT-SM11 (siehe Abb. 3) hergestellt werden. Er bewirkt in S. carnosus eine ungeminderte Sau3AI-Methylaseaktivität. Umtransformierungs-Experimente in E. coli ergaben jedoch eine deutliche Interferenz der Sau3AI-Methylase-Aktivität mit E. coli Enzymen, was die direkte Klonierung der ursprünglichen Sau3AI-Methylase in E. coli verhindert.

Überraschenderweise konnte trotzdem ein Methylase-positiver E. coli Klon erhalten werden. Dies gelang durch eine gezielte Mutation im Methylase-Gen, bei der nur eine Base ausgetauscht wurde, so daß ein verändertes Gen erhalten wurde, welches mit dem ursprünglichen Gen unter stringenten Bedingungen hybridisiert. Ein Plasmid, welches das durch Mutation veränderte Gen enthält, läßt sich ohne Verlust der Methylase-Aktivität zwischen E. coli und S. carnosus hin- und hertransferieren.

Das aktive mutierte Methylasegen konnte auch als SacI/XbaI-Fragment in die multiple Klonierungsstelle von pUC18 eingebaut werden und erfolgreich in verschiedene E. coli K12 Stämme transformiert werden.

Bei dem in E. coli aktiven mutierten Methylasegen wurde das Cytosin an Position 2134 der DNA-Sequenz SEQ ID NO:1 durch gerichtete Mutagenese in ein Thymidin umgewandelt. Diese Mutation bewirkt eine Umwandlung eines Arginincodons (CGA) in ein opal-Stopcodon (TGA). Es handelt sich also um eine Nonsense-Mutation im Leserahmen des sau3AIM-Gens, die eine Verringerung der Genexpression hervorruft, aber die Unverträglichkeit der Sau3AI-Methylase mit E. coli soweit reduziert, daß das mutierte Gen problemlos in E. coli klonierbar ist. Ein gewisser Methylasespiegel ist überraschenderweise in mit dem mutierten Gen transformierten Zellen noch vorhanden, da das Gen eine Resistenz von E. coli DNA gegen Sau3AI-Verdau bewirkt.

Ein weiterer Gegenstand der vorliegenden Erfindung ist eine DNA, die für ein Protein mit einer in E. coli exprimierbaren Sau3AI-Methylase-Aktivität kodiert. Vorzugsweise besitzt diese DNA eine im Vergleich zum Wildtyp modifizierte Nukleotidsequenz, so daß ein durch sie über Transkription und Translation entstehendes Sau3AI-Methylase-Protein eine im Vergleich zum Wildtyp verringerte Methylase-Gesamtaktivität besitzt. Ein solches modifiziertes Gen kann sich dadurch vom Wildtyp unterscheiden, daß mittels rekombinanter DNA-Technologie eine oder mehrere Mutationen innerhalb oder/und außerhalb (z.B. Promotor oder ribosomale Bindungsstelle) des kodierenden Bereichs des Sau3AI-Methylasegens eingeführt werden, welche eine Verträglichkeit des Gens im jeweiligen Wirtsorganismus, vorzugsweise in E. coli hervorrufen.

Allgemein geeignet im Sinne der Erfindung sind Modifikationen (d.h. Substitutionen, Deletionen oder Einbau von zusätzlichen Basen) im Bereich des Sau3AI-M-Gens (d.h. im Gen oder in dessen Nachbarschaft), welche durch Verringerung der Expressionshöhe oder/und der spezifischen Aktivität des Proteins eine Reduzierung der Methylase-Gesamtaktivität in der Zelle bewirken. Darunter fallen z.B. auch Deletionen größerer DNA-Fragmente der kodierenden Sequenz oder/und Fusionen mit anderen Proteinen oder Polypeptiden, wodurch die spezifische Methylase-Aktivität des Proteins verringert wird.

Vorzugsweise sind dabei Methylase-Mutanten geeignet, die im kodierenden Bereich des Gens, besonders bevorzugt innerhalb der ersten 1000, am meisten bevorzugt innerhalb der ersten 500 Basen ein Stopcodon enthalten, dem vorzugsweise mindestens ein A-Rest folgt. Geeignete Stopcodons sind z.B. Opal-Stopcodons. Solche Mutationen bewirken eine Senkung der Expressionsrate des Methylase-Gens in E. coli um 90 bis 95%.

Die Aufgabe der vorliegenden Erfindung war eine verbesserte Expression der Sau3AI-Endonuklease. Ein weiterer Gegenstand der vorliegenden Erfindung ist somit auch ein rekombinanter Vektor, der eine oder mehrere Kopien einer erfindungsgemäßen, insbesondere für das Sau3AIR-Gen kodierenden DNA enthält. Vorzugsweise ist der erfindungsgemäße rekombinante Vektor zur Vermehrung in prokaryontischen Organismen geeignet. Es kann sich dabei um einen extrachromosomal vorliegenden Vektor (z.B. Plasmid) oder um einen, sich in das Wirtsgenom integrierenden Vektor (z.B. Bakteriophage λ) handeln. Vorzugsweise ist der erfindungsgemäße Vektor ein Plasmid. Besonders bevorzugt enthält der erfindungsgemäße rekombinante Vektor einen in E. coli aktiven Replikationsursprung, d.h. er ist in E. coli vermehrbar.

Am meisten bevorzugt ist ein rekombinanter Vektor, auf dem sich das Sau3AI-Endonukleasegen unter Kontrolle eines regulierbaren Promotors befindet. Dabei muß der eigene Promotor des Sau3AIR-Gens inaktiv sein, z.B. durch eine Deletion bis auf wenige Nukleotide vor der ribosomalen Bindungsstelle. Der regulierbare Promotor muß sehr "dicht" sein, d.h. unter reprimierenden Bedingungen darf möglichst keine Transkription stattfinden, da die Sau3AI Endonuklease ein für die Zelle sehr toxisches Protein darstellt. Geeignete regulierbare und "dichte" Promotoren sind z.B. der fdhF-Promotor (EP-A 0 285 152), der pdoc-Promotor in E. coli JM83 mit lysogenem Phagen Lambda (O'Connor und Timmis, J. of Bacteriol. 169 (1987), 4457-4462) oder ein T7-Promotor, der sich auf einem "high copy"-Plasmid (z.B. pUC 18) befinden kann, in Verbindung mit einem chromosomalen T7-RNA-Polymerasegen unter Kontrolle des induzierbaren lac UV5 Promotors und dem T7-Lysozymgen (T7-Lysozym ist ein spezifischer Inhibitor für die T7-RNA-Polymerase) auf einem weiteren Plasmid, z.B. pLys5 (Studier und Moffat, J. Mol. Biol. 189 (1986), 113 ff; Moffat und Studier, Cell 49 (1987), 221-227).

Ein erfindungsgemäßer Vektor zur rekombinanten Expression der Sau3AI-Endonuklease enthält vorzugsweise auch mindestens einen Transkriptionsterminator 5'-stromaufwärts des Sau3AIR-Gens, um ein "Durchlesen" von einem 5'-stromaufwärts gelegenen Gen weitestgehend zu verhindern. Als gut geeignet hat sich z.B. der λT₀-Terminator (Schwarz et al., Nature 272 (1978), 410-414) erwiesen. Andere starke Terminatoren (die einem Fachmann geläufig sind) können jedoch auch eingesetzt werden.

Ein weiterer Gegenstand der vorliegenden Erfindung ist ein Mikroorganismus, der mit einer erfindungsgemäßen DNA oder einem oder mehreren erfindungsgemäßen rekombinanten Vektoren transformiert ist.

Dabei sind natürlich einerseits Mikroorganismen geeignet, die ursprünglich, d.h. vor der Transformation mit dem oder den erfindungsgemäßen Vektoren nicht die Gene des Sau3AI-R-M-Systems enthalten. Andererseits kann man hierfür auch einen S. aureus 3AI-Produzentenstamm, der üblicherweise zur Gewinnung der Sau3AI-Endonuklease verwendet wird und bereits das Sau3AI-R-M-System enthält, mit einem erfindungsgemäßen, für Staphylokokken geeigneten Vektor (z.B. pSEM7) transformieren. Auf letztgenannte Weise ist eine deutliche Verbesserung der R-Sau3AI-Expression möglich, wobei auch die beim Zellaufschluß von Staphylokokken auftretenden Probleme verringert werden.

Als Mikroorganismus, der ursprünglich kein Sau3AI-R-M-System enthält, ist z.B. auch der Zwischenklonierungsstamm S. carnosus, transformiert mit pSEM7 (DSM 5875) geeignet.

Als Wirtsorganismus für einen erfindungsgemäßen Vektor bevorzugt sind jedoch Mikroorganismen der Stämme E. coli, Pseudomonas und Bacillus, insbesondere E. coli. Bei Verwendung eines derartigen Mikroorganismus muß beachtet werden, daß das Sau3AI-R-Gen ohne schützende Methylase für den Organismus extrem toxisch ist.

In einer bevorzugten Ausführungsform der vorliegenden Erfindung ist der Mikroorganismus daher mit einem rekombinanten Vektor transformiert, welcher eine DNA enthält, die sowohl ein für den jeweils verwendeten Mikroorganismus verträgliches Protein mit einer Sau3AI-Methylase-Aktivität als auch für ein Protein mit einer Sau3AI-Endonuklease-Aktivität kodiert. Auf diese Weise ist es möglich, eine rekombinante Expression des Sau3AI-Restriktionsenzyms im transformierten Organismus zu erreichen, da gleichzeitig neben dem Sau3AI-Endonuklease-Gen eine aktive Sau3AI-Methylase vorliegt, welche die DNA des Wirtsorganismus vor der Spaltung schützt. Verwendet man einen Mikroorganismus wie z.B. E. coli, in welchem das Methylase-Wildtyp-Gen nicht exprimierbar ist, so verwendet man ein geeignetes mutiertes Methylase-Gen (siehe oben).

In einer weiteren bevorzugten Ausführungsform ist der Mikroorganismus mit zwei miteinander kompatiblen Vektoren transformiert, d.h. der Mikroorganismus enthält zwei Vektoren mit unterschiedlichen Origins of Replication, die auf Dauer in der transformierten Zelle zusammen vorliegen können. Einer dieser Vektoren enthält eine DNA, die ein für den jeweils verwendeten Mikroorganismus verträgliches Protein mit einer Sau3AI-Methylase-Aktivität kodiert. Somit liegt die DNA des transformierten Mikroorganismus bereits in methylierter Form vor, wenn der Mikroorganismus mit dem zweiten Vektor transformiert wird, der mit dem ersten Vektor kompatibel ist und der eine DNA enthält, die für ein Protein mit einer Sau3AI-Endonuklease-Aktivität kodiert. Auf diese Weise ist eine rekombinante Expression des Sau3AI-Restriktionsenzyms in transformierten Mikroorganismen, z.B. E. coli möglich. Die Auswahl geeigneter kompatibler Vektoren bleibt dem Fachmann überlassen, z.B. kann ein für E. coli geeignetes Vektorsystem einen Vektor mit ColE1 Origin of Replication (wie z.B. pBR322) und einen zweiten Vektor mit einem p15A Origin of Replication (Chang und Cohen, J. Bacteriol. 134 (1978), 1141-1156) enthalten.

Eine generell bevorzugte Möglichkeit zur Transformation des Sau3AI-R-Gens in einen Wirtsorganismus besteht darin, einen Vektor zu verwenden, auf dem sich das Sau3AI-Gen unter Kontrolle eines regulierbaren "dichten" Promotors (siehe oben) befindet. Die Verwendung eines regulierbaren dichten Promotors ist ohne Anwesenheit eines Methylase-Gens zwingend notwendig, jedoch auch beim Vorhandensein eines für die Zelle verträglichen Methylase-Gens empfehlenswert. So kann z.B. zur weiteren Verringerung der Toxizität der Sau3AI-Endonuklease für die Zelle der Mikroorganismus zusätzlich mit einem kompatiblen Vektor transformiert werden, welcher ein für den jeweiligen Mikroorganismus verträgliches, gegebenenfalls mutiertes Methylase-Gen enthält.

Weiterhin beinhaltet die Erfindung auch ein Verfahren zur Gewinnung einer DNA, die für ein Protein mit einer Sau3AI-Methylase-Aktivität oder/und für ein Protein mit einer Sau3AI-Endonuklease-Aktivität kodiert, bei dem man

(1) eine Genbank aus chromosomalen DNA-Fragmenten eines Ausgangsorganismus, welcher das Sau3AI-Restriktions-Modifikationssystem besitzt, durch Transformation eines, mit dem Ausgangsorganismus nahe verwandten Organismus herstellt, welcher ursprünglich kein Sau3AI-Restriktions-Modifikationssystem besitzt,

(2) Klone des transformierten Organismus auf Sau3AI-Methylase-Aktivität testet und

(3) positive Klone mit Sau3AI-Methylase-Aktivität isoliert und daraus solche Klone isoliert, welche zusätzlich ein Gen enthalten, das für ein Protein mit einer Sau3AI-Endonuklease-Aktivität kodiert.

Auf diese Weise ist es möglich, die Gene für das Sau3AI-R-M-System zu isolieren. Vorzugsweise verwendet man als Ausgangsorganismus Staphylococcus aureus 3AI und als mit dem Ausgangsorganismus nahe verwandten Organismus Staphylococcus carnosus, Staphylococcus xylosus C2A oder von S. aureus 3AI verschiedene Staphylococcus aureus Stämme. Besonders bevorzugt ist S. carnosus, DSM 5875 (ohne das Plasmid pSEM7). Zur Herstellung der Genbank des Ausgangsorganismus verwendet man einen geeigneten Vektor, der im mit dem Ausgangsorganismus nahe verwandten Organismus replizierbar ist. Die Herstellung einer S. aureus Genbank in einem geeigneten Vektor und die Transformation des mit S. aureus nahe verwandten Organismus mit dem rekombinanten Vektor kann mittels dem Fachmann auf dem Gebiet der Molekularbiologie an sich bekannten Techniken geschehen. Erfindungswesentlich ist, daß der nahe verwandte Organismus kein Sau3AI-Restriktions-Modifikationssystem besitzt, da sonst keine Selektion auf positive Klone möglich wäre.

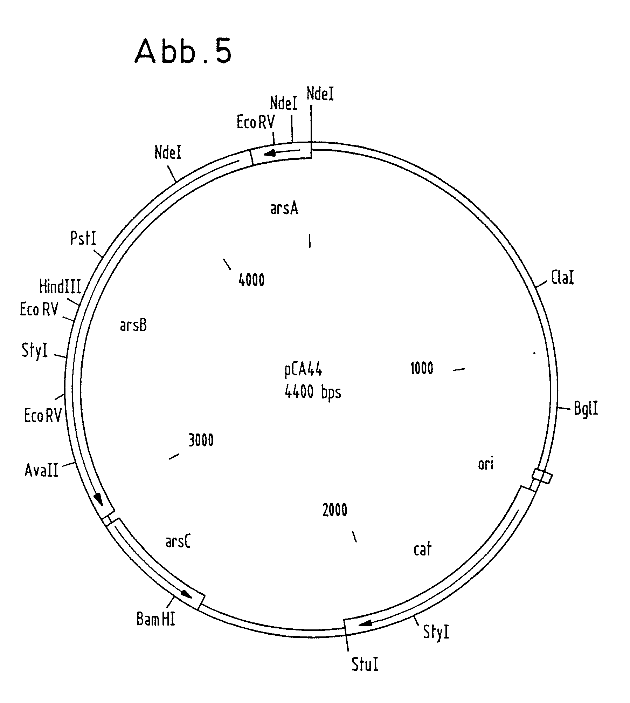

Als Klonierungsvektor für das erfindungsgemäße Verfahren kann z.B. das Plasmid pCA44 (Abb. 5), ein 4,4 kb großes ScaI/PvuII-Deletionsderivat von pCA43 (Kreutz und Götz, Gene 31 (1984), 301-304) dienen, in das die partiell geschnittene chromosomale DNA von S. aureus 3AI eingebaut wurde. Eine durch Transformation von S. carnosus, DSM 5875 (ohne Plasmid pSEM7), als Protoplasten mit dem Klonierungsvektor erhaltene Genbank wird dann auf Sau3AI Methylase-Aktivität getestet. Dazu inkubiert man vorzugsweise Plasmid-Präparationen aus den Klonen der Genbank mit käuflichem Sau3AI und untersucht die Ansätze elektrophoretisch. Ein Klon, welcher das Sau3AI-Methylase-Gen exprimiert, ist nämlich gegenüber einem Restriktionsschnitt von Sau3AI resistent. Methylase-positive Klone werden isoliert und nochmals getestet.

Auf diese Weise wurden zwei Klone mit Methylase-Aktivität erhalten. Im Rohextrakt dieser beiden Klone konnte auch Sau3AI-Endonuklease-Aktivität nachgewiesen werden. Aus beiden Klonen wurden die rekombinanten Plasmide isoliert und von dem kleineren der beiden eine Restriktionskarte angefertigt. Dieses 14,3 kb große Plasmid pSEM7 (Abb. 1) enthält beide Gene des Sau3AI-RM-Systems auf einer 9,6 kb großen Insertion.

Von der Insertion des Plasmid pSEM7 wurde eine Restriktionskarte bestimmt und die Lokalisation des Endonuklease- und Methylasegens wurde durch Konstruktion verschiedener Deletionen und Subklone festgestellt (Abb. 4). Auf diese Weise wurde nicht nur die Lokalisation der R-M-Gene auf dem Plasmid pSEM7 festgestellt, sondern es wurde auch gefunden,

daß beide Gene ihren eigenen Promotor besitzen. Auf die oben beschriebene Weise kann man somit die für das Sau3AI-R-M-System kodierende DNA erhalten. Die vorliegende Erfindung umfaßt weiterhin ein Verfahren zur Gewinnung einer erfindungsgemäßen rekombinanten DNA, die auch in einem Zielorganismus exprimierbar ist, in dem an sich keine Expression des Sau3AI-Methylase-Wildtyp-Gens möglich ist. Dies kann dadurch geschehen, daß man gezielt mittels bekannter Techniken (z.B. in vitro Mutagenese) Mutationen außerhalb (Promotor, ribosomale Bindungsstelle) oder/und innerhalb des kodierenden Bereichs des Sau3AI-Methylase-Gens einführt, welche eine Expression der Sau3AI-Methylase im Zielorganismus erlauben. Welcher Art diese Mutationen sind, wurde bereits weiter oben im einzelnen beschrieben.

Auf diese Weise erhält man ein Sau3AI-Methylasegen, welches in einem gewünschten Zielorganismus, vorzugsweise E. coli, exprimierbar ist. Besonders bevorzugt besitzt ein solches Gen im Vergleich zum Wildtyp-Gen ein Opal-Stopcodon im kodierenden Bereich, vorzugsweise in den ersten 1000, besonders bevorzugt in den ersten 500 Basen des Methylase-Gens, dem direkt danach ein A-Rest folgt.

Die Erfindung beinhaltet auch ein Verfahren zur Gewinnung eines rekombinanten Proteins mit Sau3AI-Endonuklease-Aktivität, indem man einen mit einem oder mehreren erfindungsgemäßen rekombinanten Vektoren transformierten Mikroorganismus, wobei sich auf einem der Vektoren mindestens eine Kopie eines Sau3AI-Endonuklease-Gens befinden muß, in einem für den jeweiligen Mikroorganismus geeigneten Medium unter jeweils geeigneten Expressionsbedingungen kultiviert und das Protein aus dem Zellextrakt oder Medium anreichert.

Als Wirtsorganismus verwendet man bei diesem Verfahren vorzugsweise einen prokaryontischen Mikroorganismus. Dabei kann es sich einerseits um einen Mikroorganismus handeln, welcher ursprünglich kein Sau3AI-Restriktions-Modifikations-System besitzt. Als Beispiele wären hier E. coli, Bacillus oder Pseudomonas, besonders bevorzugt E. coli zu nennen. Es ist jedoch eine Gewinnung des Sau3AI-Restriktionsenzyms auch aus Staphylococcus carnosus, DSM 5875 (ohne pSEM7), der mit einem Vektor transformiert ist, welcher das Sau3AI-R-M-System enthält (z.B. pSEM7), möglich. Für das Verfahren zur Gewinnung der Sau3AI-Endonuklease aus einem rekombinanten Wirtsorganismus ist es besonders bevorzugt, das Endonuklease-Gen unter Kontrolle eines regulierbaren dichten Promotors einzuführen, gegebenenfalls zusammen mit einem für den jeweiligen Wirtsorganismus verträglichen Methylase-Gen. Das Methylase-Gen kann sich dabei auf dem gleichen Vektor wie das Endonuklease-Gen befinden.

Es kann jedoch auch auf einem weiteren Vektor (intrachromosomal oder extrachromosomal) vorliegen. Durch Expressionskontrolle mit Hilfe eines regulierbaren dichten Promotors oder/und durch Schutz der zellulären DNA vor Spaltung in Anwesenheit eines Methylase-Gens gelingt es, rekombinante Sau3AI-Endonuklease aus transformierten Wirtszellen zu gewinnen.

Bei Verwendung eines Sau3AI-Endonuklease-Gens unter Kontrolle eines regulierbaren dichten Promotors kultiviert man die Mikroorganismen zunächst zweckmäßig in einem reprimierten Zustand (d.h. keine Expression des Sau3AIR-Gens findet statt) und führt dann einen Induktionsschritt durch, welcher innerhalb kurzer Zeit eine starke Expression der Sau3AI-Endonuklease bewirkt.

Man kann jedoch auch andererseits den üblicherweise zur Gewinnung der Sau3AI-Endonuklease verwendeten S. aureus Stamm 3AI mit einem Vektor transformieren, welcher das Sau3AI-Endonuklease-Gen und gegebenenfalls auch das Sau3AI-Methylasegen enthält. Ein solcher transformierter S. aureus 3AI Stamm ist ebenfalls zur Gewinnung der Sau3AI-Endonuklease gut geeignet, da durch die erhöhte Expression des Plasmid-kodierten Endonukleasegens die Aufreinigung des Enzyms erheblich erleichtert wird.

Durch das erfindungsgemäße Verfahren ist somit eine hohe Expression des Sau3AI-Restriktionsenzyms in verschiedenen Organismen möglich, welche, wie z.B. E. coli, nicht pathogen und gut aufschließbar sind und keine störenden Nebenaktivitäten produzieren. Auf diese Weise gelingt durch das erfindungsgemäße Verfahren eine ganz erhebliche Kostenreduzierung für die Herstellung des für die Molekularbiologie bedeutsamen Restriktionsenzym Sau3AI.

Somit ist ein Gegenstand der vorliegenden Erfindung auch ein rekombinantes Protein mit Sau3AI-Endonuklease-Aktivität, welches aus einem Organismus erhalten wird, der mit einem oder mehreren erfindungsgemäßen rekombinanten Vektoren transformiert wurde und gegebenenfalls ursprünglich kein Sau3AI-Restriktions-Modifikations-System enthält.

Schließlich beinhaltet die vorliegende Erfindung auch noch ein Protein mit Sau3AI-Methylase-Aktivität, das von einer erfindungsgemäßen DNA kodiert wird. Die Gewinnung einer Sau3AI-Methylase ist aus S. carnosus, DSM 5875 (ohne Plasmid) möglich, der mit einem geeigneten, das Methylase-Gen enthaltenden Vektor wie pTSM1 transformiert ist. Eine Gewinnung der Wildtyp-Sau3AI-Methylase aus E. coli ist möglich, wenn sich das dafür kodierende Gen unter Kontrolle eines regulierbaren dichten Promotors befindet. Die Gewinnung einer modifizierten, für E. coli verträglichen Methylase ist durch Subklonierung auf übliche Weise möglich.

Schließlich beinhaltet die Erfindung auch ein Reagenz zur Restriktionsspaltung von DNA, das eine rekombinante Sau3AI-Endonuklease enthält, die aus einem mit einem oder mehreren erfindungsgemäßen Vektoren transformierten Organismus gewonnen wurde.

Folgende Mikroorganismen und Plasmide wurden bei der Deutschen Sammlung für Mikroorganismen, Mascheroder Weg 1b, D-3300 Braunschweig, hinterlegt:

1) pBN 208 mit der Hinterlegungsnummer DSM 4069P am 27. März 1987

2) Staphylococcus carnosus, transformiert mit dem Plasmid pSEM7 mit der Hinterlegungsnummer DSM 5875 am 17. April 1990

Folgende Beispiele sollen die Erfindung zusammen mit dem Sequenzprotokoll und den Abbildungen 1 bis 7 verdeutlichen.

Es zeigen:

| | |
|---|---|
| SEQ ID NO: | 1 die Nukleotid- und Aminosäuresequenz der Gene für das Sau3AI-Restriktions-Modifikations-System, |
| Abb. 1 | das Plasmid pSEM7, , |
| Abb. 2 | das Plasmid pTSM1, |
| Abb. 3 | das Plasmid pBT-SM11, |
| Abb. 4 | die Herstellung von Deletionen und Subklonen zur Charakterisierung der Insertion von pSEM7, |
| Abb. 5 | das Plasmid pCA44, |
| Abb. 6 | das Plasmid pUSM 1, |
| Abb. 7 | die Konstruktion eines Sau3AI-Expressionsvektors für E. coli. |

**Beispiel** 1

Klonierung des Sau3AI-R-M-Systems

1.1 Herstellung von S. aureus DNA-Fragmenten und Ligation in einen Vektor

Für die Anzucht von Staphylokokken wurde BM-Medium mit folgender Zusammensetzung verwendet:

| | | |
|---|---|---|
| Trypton | 10 | g |
| Hefeextrakt | 5 | g |
| NaCl | 5 | g |
| Glucose | 1 | g |
| $K_2 HPO_4$ | 1 | g |
| $H_2 O$dest | ad 1 | l |
| pH 7,2 | | |

Für die Anzucht von pCA44, pSEM7 in S. carnosus, DSM 5875 (ohne Plasmid) wird dem Medium 10 μg/ml Chloramphenicol zugegeben. Für die Anzucht von pTSM1, pBT-SM11 in S. carnosus, DSM 5875 (ohne Plasmid) wird dem Medium 20 μg/ml Tetracyclin zugegeben. Plasmid-DNA und chromosomale DNA von S. aureus wurde nach dem Klar-Lysat-Verfahren (Novick und Bouanchand, Ann. N.Y. Acad. Sci. 182 (1971), 279-291) isoliert.

Durchführung der Plasmid-Präparation:

- 1 bis 1,5 l BM-Medium in 5 l-Erlenmeyer-Kolben (mit entsprechendem Antibiotikum) mit Vorkultur animpfen und bei 37°C bis zum Ende der exponentiellen Phase (ca. 12 bis 18 Stunden) inkubieren;
- Zellen abzentrifugieren und mit 40 ml EDTA-Waschlösung (0,15 mol/l EDTA, pH 8,0) waschen.
- Resuspendieren in 60 ml NaCl-Puffer (0,05 mol/l Tris/HCl; 2,5 mol/l NaCl; 0,05 mol/l EDTA; pH 8,0) und in 250 ml Erlenmeyerkolben überführen;
- 1 bis 1,5 ml Lysostaphin (Sigma)-Lösung (0,5 mg/ml) zugeben und bei 37°C unter leichtem Schütteln inkubieren, bis die Suspension eine schleimig, viskose Konsistenz annimmt (nach 15 bis 25 Minuten);
- mit 60 ml eiskaltem Lysepuffer (0,05 mol/l Tris/NaOH; 0,3 mol/l EDTA; 0,5 % Brij 58, 0,04 % Na-desoxy-cholat; pH 8,0) vermischen und eine Stunde auf Eis stellen.
- Zentrifugation bei 27.500 g, 4°C, 20 Minuten;
- Überstand vorsichtig abgießen (Pellet oft sehr weich) und mit 30 ml 50 % PEG (PEG 6000) 2 Stunden bei 4°C fällen;
- Zentrifugation bei 8000 g, 4°C, 20 Minuten;

- Überstand abgießen und das glasig, opake Pellet sorgfältig in 10 ml TE-Puffer (10 mmol/l Tris, 1 mmol/l EDTA, pH 8) lösen;
- Zugabe einer Spatelspitze Proteinase-K (Serva). Inkubation bei 37° C 15 Minuten unter leichtem Schütteln;
- Reinigung durch CsCl-Dichtegradientenzentrifugation.

Als zweckmäßigste Methode zur Präparation von chromosomaler DNA aus Staphylokokken hat sich eine leichte Abwandlung des Plasmidisolierungsprotokolls erwiesen. Der einzige Unterschied zur oben aufgeführten Methode besteht darin, die Suspension während der einstündigen Lyse ab und zu gut durchzuschütteln, um so die Freisetzung hochmolekularer DNA aus den Zellen zu ermöglichen und die Ausbeute an chromosomaler DNA zu erhöhen. Nach der Ethanolfällung sollte die DNA auf einen Glasstab aufgewickelt und anschließend in TE-Puffer gelöst werden. Das Abzentrifugieren ist wegen der schlechten Löslichkeit verklumpter, chromosomaler DNA zu vermeiden.

Die chromosomale S. aureus 3AI-DNA wurde partiell mit MboI gespalten. Es wurden Fragmente im Größenbereich von 7 bis 15 kb über ein Agarosegel angereichert. Diese Fragmente wurden in den, mit BamHI geschnittenen und mit alkalischer Phosphatase behandelten Vektor pCA44 (Abb. 5), einem 4,4 kb großen ScaI/PvuII-Deletions-Derivat von pCA43 (Kreutz und Götz (1984), supra) ligiert.

1.2 Herstellung einer S. aureus-Genbank in Staphylococcus carnosus, DSM 5875 (ohne Plasmid)

Die Transformation von S. carnosus mit dem ligierten Vektor aus 1.1 wurde durch Polyethylenglykol induzierte Protoplasten-Fusion durchgeführt, wie bei Götz und Schumacher (FEMS Letts. 40 (1987), 285-288) beschrieben.

Durchführung:

a) Herstellung von Protoplasten
- 6x 300 ml BM-Medium mit je 1,5 ml einer 12 Stunden-Kultur von Staphylococcus carnosus, DSM 5875 (ohne Plasmid) beimpfen;
- Bis zu einer OD578 von 0,4 bis 0,5 wachsen lassen;
- Zellen in sterilen, verschraubbaren Plastikbechern bei 3300 g, 4° C, 20 Minuten abzentrifugieren;
- Die Zellsedimente in je 30 ml SMMP (siehe Götz und Schumacher (1987), supra) suspendieren und in sterile 100 ml-Kölbchen überführen;
- Zugabe von 20 µl sterilfiltrierter Lysostaphinlösung (0,5 mg/ml);
- Kölbchen 12 bis 16 Stunden bei 30° C stehen lassen;
- Protoplastierung im Lichtmikroskop kontrollieren (Zellen sollten vereinzelt, vergrößert und nicht lysiert sein);
- Protoplasten in sterilen Plastik-Schraubdeckelröhrchen (40 ml) bei 4000 g und Raumtemperatur 25 Minuten zentrifugieren;
- Zellen mit 5 ml SMMP waschen;
- Sediment in 2 ml SMMP lösen und in 300 µl-Portionen in sterile Schraubdeckelröhrchen aufteilen; Lagerung nur bei -70° C möglich.

b) Protoplasten-Transformation
- 300 µl Protoplastensuspension langsam auftauen lassen;
- Transformierende DNA-Lösung zugeben;
- Sofort 2 ml PEG 6000 (40%) in SMM (2x SMM: 1 mol/l Saccharose, 40 mmol/l Maleinsäure, 40 mmol/l MgCl2, pH 6,8) zugeben und vorsichtig mischen;
- nach 2 Minuten Transformation durch Zugabe von 7 ml SMMP (8 Teile 2x SSM, 2 Teile 4x PAB (Difco Antibiotic Medium), 370 g/l, 0,5 Teile 5 % BSA) stoppen;
- Abzentrifugieren (4000 g, Raumtemperatur, 25 Minuten);
- Sediment durch vorsichtiges Schütteln in 1 ml SMMP resuspendieren;
- Je 100 bis 200 µl der Protoplastensuspension auf DM-3 Platten (Götz und Schumacher, FEMS Letters 40, 1987, 285-288) ausplattieren; bei Transformation mit intakten Plasmiden verdünnen ($10^3$- bis $10^6$-fach);
- Platten zur Regeneration der Zellwände und zur phänotypischen Expression der Resistenzmerkmale 3 bis 4 Stunden bei 37° C inkubieren;
- Platten mit je 3 ml hemmstoffhaltigem CY-3 Agar (Götz und Schumacher, FEMS Letters 40, 1987, 285-288) überschichten; Hemmstoff 10fach konzentriert zugeben (Chloramphenicol 100 µg/ml, Tetrcyclin 200 µg/ml; wegen Verdünnung durch Diffusion in 30 ml DM-3 Agar);
- 2 Tage bei 37° C inkubieren.

1.3 Isolierung von M• Sau3AI und R• Sau3AI-aktiven Klonen

Die DNA-Isolierung aus S. carnosus wurde in großem Maßstab nach Götz et al., (Plasmid 9 (1983), 126-137), die Minipräparationen wurden im wesentlichen nach dem alkalischen Lysis Standardverfahren nach Birnboim und Doly (Maniatis et al. (1982), Molecular Cloning. A Laboratory Handbook) durchgeführt, abgesehen davon, daß 8 bis 12 μg/ml Lysostaphin 10 Minuten lang bei 37° C mit den Zellen vor der Lyse mit NaOH/SDS inkubiert wurden.

Die M* Sau3AI-Aktivität von rekombinanten S. carnosus-Klonen wurde durch gleichzeitiges Testen von jeweils 5 Klonen bestimmt, wobei die isolierten Plasmide mit Sau3AI geschnitten wurden und die gespaltene DNA durch Agarosegel-Elektrophorese untersucht wurde. Die DNA aus M* Sau3AI-exprimierenden Klonen war gegenüber Spaltung mit kommerzieller Sau3AI-Endonuklease resistent. Die Klone aus den M-positiven Ansätzen wurden getrennt und einzeln erneut getestet. Die M-aktiven Klone wurden auf R-Aktivität durch Inkubation von 1 μl des Rohextrakts (hergestellt durch zweimalige Behandlung der Zellen in einer French-Press mit 16000 psi) mit 1 μg pBR322 eine Stunde lang bei 37° C getestet. Nach Agarosegel-Elektrophorese wurden die Restriktionsmuster untersucht. pBR322, das mit kommerziellem Sau3AI gespalten wurde, diente als Kontrolle.

Zwei von ungefähr 800 getesteten Transformanten zeigten Resistenz gegenüber Sau3AI-Spaltung aufgrund der Expression eines klonierten Sau3AI-Methylasegens. Diese beiden rekombinanten Plasmide pSEM7 und pSEM11 zeigten beide dieselbe Resistenz gegenüber Sau3AI-Spaltung wie die chromosomale DNA von S. aureus 3AI. Beide Klone zeigten sowohl M* Sau3AI-Aktivität als auch R* Sau3AI-Aktivität.

1.4 Charakterisierung und Sequenzanalyse der positiven Klone

Das kleinere rekombinante Plasmid pSEM7 (Abb. 1) enthält eine 9,6 kb große Insertion. Von diesem Plasmid wurde eine Restriktionskarte hergestellt und die Lokalisation des Endonuklease- und des Methylasegens durch Herstellung verschiedener Deletionen und Subklone bestimmt (Abb. 4). Die BcII/BcII und die NruI/HpaI-Deletionen hatten keinen Einfluß auf die R-M-Aktivität, während die NciI/HpaI und die NruI/ScaI-Deletionen zum Verlust der R-Aktivität bzw. beider Aktivitäten führte. Das Sau3AI-Methylasegen wurde auf einem 3,66 kb großen EcoRV-Fragment in die SmaI-Stelle des Plasmids pT181mcs kloniert, einem Derivat von pT181 (Khan und Novick, Plasmid 10 (1983), 251-259), das das PvuII-Fragment mit der multiplen Klonierungssequenz (mcs) von pUC18 in der NdeI-Stelle insertiert enthält. Das resultierende Plasmid pTSM1 ist 8,4 kb groß und trägt neben dem aktiven Methylasegen noch ein kurzes inaktives Fragment des Endonukleasegens (Abb. 2).

Die Nukleotidsequenz der Gene des Sau3AI-R-M-Systems wurde nach dem Didesoxy-DNA-Sequenzierverfahren nach Sanger bestimmt. Das Ergebnis der Sequenzanalyse ist im Sequenzprotokoll (SEQ ID NO:1) dargestellt.

**Beispiel 2**

Gewinnung von in E. coli exprimierbaren Sau3AI-Methylase-Genen

Durch Insertion eines 2,3 kb Fragments (EcoRI/PvuII, stumpfe Enden durch Klenow-Behandlung) des E. coli Vektors pBR322 (mit Replikationsorigin und ß-Lactamasegen) in die SalI-Schnittstelle (ebenso stumpfe Enden durch Klenow-Behandlung) von pTSM1 (Abb. 2, Beispiel 1.4) konnte der S. carnosus/E. coli Shuttle Vektor pBT-SM11 (Abb. 3) hergestellt werden. Er bewirkt in S. carnosus ungeminderte Sau3AI-Methylase-Aktivität.

Durchführung der Umklonierungsexperimente:

Der Shuttle-Vektor pBT-SM11 wurde in S. carnosus, DSM 5875 (ohne Plasmid) kloniert, isoliert und je 0,2 μg Plasmid-DNA zu Umklonierungs-Experimenten verwendet. Die E. coli-Zellen wurden nach dem CaCl₂-Verfahren kompetent gemacht. Die Transformationsversuche wurden nach standardisierten Verfahren durchgeführt:
- Von 1 ml kompetenten E. coli-Zellen wurden 200 μl Zellen zur Transformation eingesetzt.
- Je 100 μl von 1,3 ml transformierten Zellen wurden auf BM-Agarplatten ausplattiert.
  Als Teststämme wurden kommerziell erhältliche E. coli K12 Stämme (z.B. JM83, JM109) verwendet.
- Mit pBT-SM11 wurde in allen getesteten E. coli-Stämmen nur eine extrem niedrige Transformationsrate erhalten.

Durchführung der in vitro Mutagenese:

Eine Mutagenese des Sau3AI-Methylase-Gens erfolgte ausgehend vom Plasmid pTSM1 (Abb. 2). Das EcoRI-Fragment mit einem, am C-terminalen Ende deletierten Methylase-Gen wurde in M13mp19-Phagen umkloniert, wo dann eine Oligonukleotid gerichtete in vitro Mutagenese (Amersham Nr. 1523) durchgeführt wurde. Dabei wurde ein Oligonukleotid verwendet, das gegen die Region um den Cytosinrest an Position 2134 innerhalb eines Arginin-Codons (CGA) der DNA-Sequenz SEQ ID NO:1 gerichtet war. Es wurde eine Nonsense-Mutation in den Leserahmen des Sau3AI-Endonuklease-Gens eingeführt, bei der das C dieses Arginin Codons in ein T umgewandelt wurde, was die Entstehung eines Opal-Stop-Codons (TGA) zur Folge hatte. Das mutierte Genfragment wurde mit EcoRI wieder aus dem M13mp19-Vektor herausgeschnitten und wieder in das EcoRI-Plasmid-Fragment von pTSM1 zurückkloniert.

Das Stopcodon innerhalb des mutierten Methylase-Gens wird, insbesondere wenn es von einem oder mehreren Adeninresten gefolgt wird, mit einer Rate von ca. 10% der Ausgangsaktivität überlesen (Parker, Microbiol. Res. 53 (1989), 273-298). Anstelle eines Stopcodons wird ein Tryptophan eingebaut. Auf diese Weise entsteht eine mutierte Sau3AI-Methylase.

Ein, dieses mutierte Methylase-Gen enthaltendes Derivat von pBT-SM11, welches pBT-SM11/2 bezeichnet wurde, läßt sich ohne Probleme zwischen E. coli und S. carnosus hin und her transformieren und bewirkt in beiden Organismen eine Resistenz der DNA gegen Spaltung mit Sau3AI. Durch die Mutation wird also die Unverträglichkeit der Sau3AI-Methylase mit E. coli soweit reduziert, daß eine Klonierung dieses Gens in E. coli möglich ist.

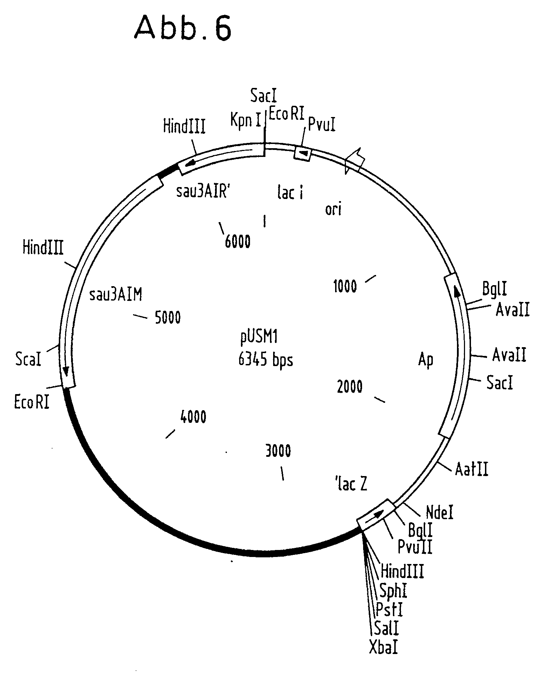

Die Insertion des 3,68 kb großen mutierten Methylasegens (SacI/XbaI-Fragment) von pBT-SM11/2 konnte in die multiple Klonierungsstelle von pUC18 (SacI/XbaI) eingebaut und erfolgreich in verschiedene E. coli K12 Stämme (z.B. JM83) kloniert werden. Der resultierende Vektor wurde pUSM1 bezeichnet und ist in Abb. 6 dargestellt.

**Beispiel** 3

Klonierung der Sau3AI-Endonuklease in E. coli JM83

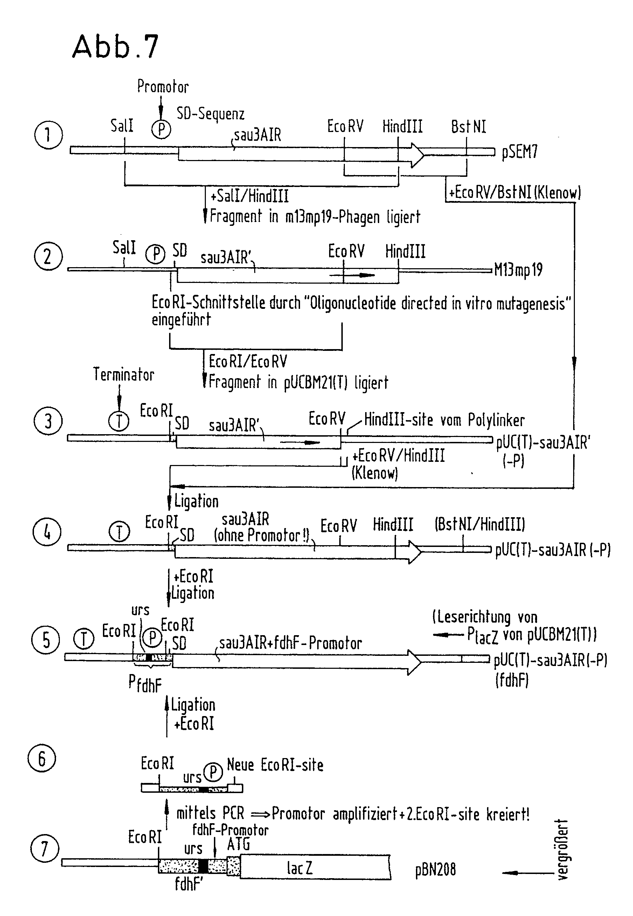

3.1 Klonierung des promotorfreien Sau3AI-Endonukleasegens hinter den reprimierbaren fdhF-Promotor
Der fdhF-Promotor:
Das fdh-Gen kodiert für das Selenpeptid der Formiat-Dehydrogenase, die zusammen mit dem Hydrogenase-Isoenzym 3 und noch unbekannten Elektronenüberträgern den Formiat-Hydrogen-Lyase-Komplex bildet.
Die fdhF-Genexpression wird durch anaerobe Bedingungen und Formiat induziert und durch geringe Mengen an Sauerstoff reprimiert. Der fdhF-Promotor (mit urs-Sequenz (upstream-regulatory-sequence) und einem kleinen Teil des fdhF-Gens) wurde auf dem Plasmid pBN208 (DSM 4069P) subkloniert (Birkmann et al., Arch. Microbiol 148 (1987), 273-298). Die DNA-Sequenzen des fdhF-Promotors und des fdhF-Gens sind beschrieben (siehe auch EP-A 0 285 152).
Von dem Plasmid pBN208 ausgehend wurde mittels Polymerase-Chain-Reaktion (PCR; mit der Taq-Polymerase) die fdhF-Promotorsequenz bis kurz vor die Shine-Dalgarno(SD)-Sequenz des fdhF-Gens amplifiziert (Innis et al., PCR Protocols. A Guide to Methods and Applications. Acad. Press Inc., 1990). Hierzu wurden zwei Oligonukleotide synthetisiert. Das erste Oligonukleotid war gegen die Sequenz stromaufwärts der EcoRI-Schnittstelle (vgl. Abb. 7, Nr. 6. und 7.) gerichtet. Das zweite Oligonukleotid war gegen die Sequenz um die SD-Sequenz des fdhF-Gens gerichtet. Es war so verändert, daß nach Amplifikation mittels PCR eine zweite EcoRI-Schnittstelle wenige Nukleotide vor der SD-Sequenz entstand. Das auf diese Weise amplifizierte, die fdhF-Promotorsequenz enthaltende DNA-Fragment wurde mit EcoRI nachgeschnitten. Auf diese Weise erhielt das DNA-Fragment auf beiden Seiten eine EcoRI-Schnittstelle.
Sau3AI-Gen ohne Promotor auf dem Vektor pUCBM21(T)-sau3AIR(-P):
Wie in Abb. 7, Nr. 1 bis 5 und aus der Plasmidkarte von pSEM7 ersichtlich, wurde ein Sau3AIR-Genfragment durch eine Doppelspaltung mit SalI/HindIII herausgeschnitten und in den ebenfalls mit SalI/HindIII-geschnittenen Phagen M13mp19 ligiert. Dort wurde dann eine "Oligonucleotide directed in vitro mutagenesis" (Amersham Nr. 1523) in einem Bereich wenige Nukleotide vor der SD-Sequenz des Sau3AIR-Genfragments in M13mp19-sau3AIR' durchgeführt. Dazu wird nach Präparation von Einzelstrang-DNA das entsprechende Oligonukleotid an die Einzelstrang-DNA hybridisiert und eine Verlängerung in 5'-3'-Richtung über das Oligonukleotid hinaus unter Verwendung von Klenow-Polymerase, Ligase und den vier Nukleotid-Triphosphaten dATP, dCTP, dGTP, dTTP durchgeführt. Die nun doppelsträngige DNA wird in E. coli JM83 transformiert. Es werden rekombinante Klone gepickt, und die Einzelstrang-DNA der darin enthaltenen M13-Phagen wird daraus isoliert. Nach bekannten Techniken wird eine DNA-Sequenzierung an der Mutagenese-Stelle durchgeführt und so der exakte Basenaustausch zur gewünschten Mutation überprüft. Dabei wurde gefunden, daß die Sequenz 5'-GCGGAAATAATTATTTAATGTTAAGAGGGG-3' (die letzten 5 Basen AGGGG definieren die SD-Sequenz) in die Sequenz 5'-GCGGAAAGAATTCTTTAATGTTAAGAGGGG-3' mutagenisiert worden war. Auf diese Weise entstand eine EcoRI-Schnittstelle (5'-GAATTC, unterstrichene Sequenz).

Durch eine EcoRI/EcoRV-Doppelspaltung der Doppelstrangform von M13mp19-sau3AIR'(mut) konnte das sau3AIR'-Genfragment ohne Promotor herausgeschnitten und in den mit EcoRI/EcoRV-geschnittenen Vektor pUCBM21(T) eingesetzt werden. Dieser Vektor enthält ein Oligonucleotid mit dem $\lambda T_o$-Terminator (E. Schwarz et al., Nature 272 (1978), 410-414) in der Pvul-Stelle von pUCBM21 (Boehringer Mannheim, Kat.Nr. 1219251). Anstelle von pUCBM21 kann man natürlich auch andere kommerzielle Vektoren mit geeigneten Klonierungsstellen z.B. die pUC-Vektoren verwenden.

Das entstandene Plasmid pUC(T)-sau3AIR'(-P) wurde mit EcoRV und HindIII geschnitten. Die HindIII-Stelle wurde anschließend durch Behandlung mit Klenow-Polymerase glattendig gemacht. Daneben wurde auch pSEM7 mit EcoRV und BstNI geschnitten, das 745 bp große DNA-Fragment isoliert, die BstNI-Stelle durch Klenow-Behandlung glattendig gemacht und das resultierende Fragment mit dem gespaltenen Vektor pUC(T)-sau3AIR'(-P) ligiert. Auf diese Weise wurde die kodierende Sequenz des sau3AIR-Gens wieder vervollständigt. Auf dem resultierenden Konstrukt besitzt das sau3AIR-Gen jedoch keinen Promotor mehr.

Bei der Konstruktion des Sau3AI-Endonuklease-Expressionsvektors muß beachtet werden, daß das promotorlose sau3AIR-Gen nicht in der selben Transkriptionsrichtung wie das lacZ-Gen in den pUC-Vektor eingebaut wird, da sonst eine, für die Zelle letale Transkription des sau3AIR-Gens vom lacZ-Promotor erfolgen würde. Weiterhin enthält der Expessionsvektor vor der Insertionsstelle des promotorlosen Sau3AIR-Gens (in Transkriptionsrichtung) einen wirksamen Terminator, um ein "Durchlesen" von vorgeschalteten Genen zu verhindern.

In die EcoRI-Schnittstelle des obigen, das sau3AIR-Gen ohne Promotor enthaltenden Vektors wurde das amplifizierte, mit EcoRI nachgeschnittene fdhF-Promotorfragment eingebaut. Der Ligationsansatz wurde in E. coli JM83 transformiert. Das entstandene high-copy-Plasmid pUC(T)-sau3AIR-P(fdhF) enthält das sau3AIR-Gen unter der Kontrolle des regulierbaren fdhF-Promotors. Unter Sauerstoffzufuhr ist der Promotor reprimiert; bei Sauerstoffmangel und Formiatzugabe ist der Promotor dereprimiert und das sau3AIR-Gen kann transkribiert und die entstehende mRNA translatiert werden. Die Konstruktion des Vektors pUC(T)-sau3AIR-P(fdhF) ist schematisch in Abb. 7 dargestellt.

3.2 Konstruktion eines kompatiblen Vektors, der das mutierte Sau3AI-Methylase-Gen enthält

Um einen vollständigen Schutz der DNA einer Zelle zu gewährleisten, die mit einem Sau3AIR-Expressionsvektor transformiert ist, wurde das nach Mutagenese gemäß Beispiel 2 nur in geringem Ausmaß exprimierte, aber dadurch in E. coli klonierbare Sau3AI-Methylasegen als Sacl/Xbal-Fragment in den zu pUC kompatiblen Vektor pACYC184 (Chang und Cohen, J.Bacteriol. 134 (1978), 1141-1156) eingebaut und in E. coli JM83, transformiert mit pUC-sau3AIR-P(fdhF), kloniert. Die in den doppelt transformierten Zellen in relativ geringen Mengen vorhandene Methylase sorgt nun durch Methylierung der Sau3AI-Schnittstellen für einen hinreichenden Schutz gegen die auch in reprimiertem Zustand in sehr geringem Maße gebildete Sau3AI-Endonuklease.

SEQ ID NO: 1

ART DER SEQUENZ: Nucleotid mit zwei codierenden Bereichen
und entsprechende Proteine
SEQUENZLAENGE: 3360 Basenpaare

MERKMALE: von 448 bis 1914 BP sau3AI R - Sequenz
von 2017 bis 3252 BP sau3AI M - Sequenz

```
AAGTCGACCT TCACCAAGAC CGAATTTTCC GCGTATACCC GCAGTACCAA ACGTTAATTT    60

ACTTTCAAAA CCTTCTCGCT GTTCAATGTC AGATTGCTGC TCATAAAAAT GTTTAACTAA    120

ACTATCATTA GCTCTTTCTA TCCATAATTC TTTATCCATT GTTGCTAAAC ATCCTTTCAA    180

AATCTCAGTT AGACTTAATA AAACATGAAA ACTAAAGCCC TTACATTTAT GTAATGAATT    240

ATAAAGAAAT ACGCCCCAAA AGTAAAAAAA CACAGCCCCA AGACAATACT TTTCACAAGT    300

ATTATATAAT AGATGTGTAT GAAAATGCAT GGAGTAGATG TAAGAGTGAT ATTCAAAATG    360

TGTAAAAAAT ATGGATAATT CTATATAATT ATATTATTGA AATTTTAAAT AGCGGAAATA    420
```

ATTATTTAAT GTTAAGAGGG GATAATT TTG GAA AGT TAT TTG ACA AAA CAA GCC    474
                                                 Met Glu Ser Tyr Leu Thr Lys Gln Ala
                                                  1              5

GTA CAT AAT CGC GCA AAA GAA GCT GTT GGT AAA AGT GTA TTA GAA TTA    522
Val His Asn Arg Ala Lys Glu Ala Val Gly Lys Ser Val Leu Glu Leu
 10              15              20              25

AAT GGT GGT GAA TCG ATT AAA CAA AGT AAG AGT TCA GTT GGT GAT GCA    570
Asn Gly Gly Glu Ser Ile Lys Gln Ser Lys Ser Ser Val Gly Asp Ala
              30                 35                40

TTT GAA AAT TGG TTT GGT AAG AAA AAA GAC AGT GAT AGT AAA CCA GAT    618
Phe Glu Asn Trp Phe Gly Lys Lys Lys Asp Ser Asp Ser Lys Pro Asp
              45               50               55

ATG GCA GAA GCT GGG GTG GAA CTT AAG GCA ACG CCA TTT AAA AAG TTG    666
Met Ala Glu Ala Gly Val Glu Leu Lys Ala Thr Pro Phe Lys Lys Leu
          60               65              70

AAA AAC GGA AAG TAT AGC TCC AAA GAA AGA TTA GTA TTA AAT ATT ATA    714
Lys Asn Gly Lys Tyr Ser Ser Lys Glu Arg Leu Val Leu Asn Ile Ile
     75               80               85

AAC TAT GAG AAA GTG GCA AAT GAA AAT TTT GAA ACT AGT AGT TTT TTA    762
Asn Tyr Glu Lys Val Ala Asn Glu Asn Phe Glu Thr Ser Ser Phe Leu
 90              95              100            105

TCT AAG AAT AAT ACT ATA GAA TTA GCT TTC TAT GAA TAT ATC AAG GGA    810
Ser Lys Asn Asn Thr Ile Glu Leu Ala Phe Tyr Glu Tyr Ile Lys Gly
            110              115            120

```
ACA CCT AGT GAT AAT TGG ATT ATT AAA GAA GCG GTG CTT TAT GAA ATG        858
Thr Pro Ser Asp Asn Trp Ile Ile Lys Glu Ala Val Leu Tyr Glu Met
            125             130             135

CAT AAA AAC CCG ATT GAT TAT GAA ATA ATT AAA CAA GAT TGG GAA ATA        906
His Lys Asn Pro Ile Asp Tyr Glu Ile Ile Lys Gln Asp Trp Glu Ile
            140             145             150

ATA AAT CAA TAT ATT AAT GAA GGA AAG GCA CAT GAA TTG AGT GAA GGT        954
Ile Asn Gln Tyr Ile Asn Glu Gly Lys Ala His Glu Leu Ser Glu Gly
    155             160             165

TTG ACA AGT TAT TTA GCG CCA TGT ACA AAG GGT GCG AAT GCT AGT TCT        1002
Leu Thr Ser Tyr Leu Ala Pro Cys Thr Lys Gly Ala Asn Ala Ser Ser
170             175             180             185

TTA AGA AAT CAG CCT TAT TCA GAC ATA AAA GCA AAG CAA AGA GCA TTT        1050
Leu Arg Asn Gln Pro Tyr Ser Asp Ile Lys Ala Lys Gln Arg Ala Phe
            190             195             200

TCT TTG AAG TCT GGG TAT ATG ACA TCT ATT TTA CGC AAA TAT GTT CTA        1098
Ser Leu Lys Ser Gly Tyr Met Thr Ser Ile Leu Arg Lys Tyr Val Leu
            205             210             215

GGT GAT GAA AAA ATA GAT TCA ATT GTC AAA GAC CCA TTT GAA ATA AAA        1146
Gly Asp Glu Lys Ile Asp Ser Ile Val Lys Asp Pro Phe Glu Ile Lys
            220             225             230

GAA AAA TCA ATA GAG GAC ATA GTC TTT GAA AAA TTT CAG CCA TAT ATA        1194
Glu Lys Ser Ile Glu Asp Ile Val Phe Glu Lys Phe Gln Pro Tyr Ile
            235             240             245

AAC TGG TCA ATC GAT AAA TTA TGC GAA CAT TTT TCT ATC AAT AAA GGT        1242
Asn Trp Ser Ile Asp Lys Leu Cys Glu His Phe Ser Ile Asn Lys Gly
250             255             260             265

GAG AAA GGT TTA AAT TAT AGA ATA GCC TCT GCC ATT TTA AAT CTA AAA        1290
Glu Lys Gly Leu Asn Tyr Arg Ile Ala Ser Ala Ile Leu Asn Leu Lys
            270             275             280

GGT AAA ACT ACT AAA AGT AAA CCA TTC CCG GAA GTT GAA GAG TTT GAA        1338
Gly Lys Thr Thr Lys Ser Lys Pro Phe Pro Glu Val Glu Glu Phe Glu
            285             290             295

AAA TCA TCT ATA GTA GTC AAA ACA GTT CAT TTT AAT AAA AAG AAT GTG        1386
Lys Ser Ser Ile Val Val Lys Thr Val His Phe Asn Lys Lys Asn Val
            300             305             310

AAT AAA GAA AGT ATG TCA TTT GGA GCT TTT AAA TTT GAA GAA CTA GCT        1434
Asn Lys Glu Ser Met Ser Phe Gly Ala Phe Lys Phe Glu Glu Leu Ala
            315             320             325

AAT GAG GAA TGG GAA GAT AGT GAA GGA TAT CCT AGT GCA CAA TGG CGA        1482
Asn Glu Glu Trp Glu Asp Ser Glu Gly Tyr Pro Ser Ala Gln Trp Arg
330             335             340             345
```

```
AAC TTT TTG TTA GAA ACA AGG TTT TTA TTT TTT GTT GTT AAA GAA GAT   1530
Asn Phe Leu Leu Glu Thr Arg Phe Leu Phe Phe Val Val Lys Glu Asp
            350             355             360

GAA GAT GGT GTA GAC ATA TTC AAA GGA ATA AAA TTT TTT AGT ATG CCT   1578
Glu Asp Gly Val Asp Ile Phe Lys Gly Ile Lys Phe Phe Ser Met Pro
            365             370             375

GAA GAA GAC ATA AAC GGA CCT GTC AAA AGA ATG TGG GAT GAT ACA GTG   1626
Glu Glu Asp Ile Asn Gly Pro Val Lys Arg Met Trp Asp Asp Thr Val
            380             385             390

AAG AAA TTA AAA GAG GGT GTC ACA TTA GAA GCT GTA CCG GAC AAA AGT   1674
Lys Lys Leu Lys Glu Gly Val Thr Leu Glu Ala Val Pro Asp Lys Ser
    395             400             405

ACA AAG GAT GGT TGG AGA ATA AAA AAT AAT TTT GTA GAT AAA AGT GAT   1722
Thr Lys Asp Gly Trp Arg Ile Lys Asn Asn Phe Val Asp Lys Ser Asp
410             415             420             425

GAT TTA ATT TGC CAT GTT AGA CCA CAC ACT AAT AAC AGA GAC TAT CGT   1770
Asp Leu Ile Cys His Val Arg Pro His Thr Asn Asn Arg Asp Tyr Arg
430             435             440             445

GGA GGA AGT AAT GCA GAT AAG CTT CCT AAA AAG ATT AAC TGG ATT AAT   1818
Gly Gly Ser Asn Ala Asp Lys Leu Pro Lys Lys Ile Asn Trp Ile Asn
            450             455             460

AGA CCT GAC TCA GAT GAT TAT TCG GAT GAG TGG ATG ACT AAA CAA AGT   1866
Arg Pro Asp Ser Asp Asp Tyr Ser Asp Glu Trp Met Thr Lys Gln Ser
            465             470             475

TTT TGG ATA AAT AAT GAC TAC ATA AAA AAG CAA GTT GAA GAT TTA TTG   1914
Phe Trp Ile Asn Asn Asp Tyr Ile Lys Lys Gln Val Glu Asp Leu Leu
            480             485             490

TAGTTAAAGT ATGTTAAAAT ATAAGATATT CTTTTAAAAT ATCGAACGAT CGTTCGTATT   1974


TTGTGTTATA ATAAGGTTGA ATTAAGTATA GGAGGTCGCC TAATG AAT AAA ATT AAA   2031
                                             Met Asn Lys Ile Lys
                                                 1

GTA GTA GAA TTG TTT GCG GGT GTA GGC GGG TTT CGT TTA GGT TTA GAA   2079
Val Val Glu Leu Phe Ala Gly Val Gly Gly Phe Arg Leu Gly Leu Glu
  5             10              15              20

AAT ACG AAA AAT GGT ATA TTT GAC ATA ACT TGG GCA AAT CAA TGG GAG   2127
Asn Thr Lys Asn Gly Ile Phe Asp Ile Thr Trp Ala Asn Gln Trp Glu
            25              30              35

CCC TCA CGA AAA ATC CAA CAT GCA TTT GAT TGT TAT AGT AAA AGA TTT   2175
Pro Ser Arg Lys Ile Gln His Ala Phe Asp Cys Tyr Ser Lys Arg Phe
            40              45              50
```

```
AAG AAC GGC ATC CAT AGT AAT AAG GAT ATT GCC CAG GTA TCT GAT GAA        2223
Lys Asn Gly Ile His Ser Asn Lys Asp Ile Ala Gln Val Ser Asp Glu
        55                  60                  65

GAA ATG GCA AAT ACT GAA GCT GAT ATG ATT GTA GGA GGA TTT CCT TGC        2271
Glu Met Ala Asn Thr Glu Ala Asp Met Ile Val Gly Gly Phe Pro Cys
        70                  75                  80

CAA GAT TAT TCA GTT GCA AGG AGT TTA AAT GGA GAA TTA GGA ATA CAA        2319
Gln Asp Tyr Ser Val Ala Arg Ser Leu Asn Gly Glu Leu Gly Ile Gln
85                  90                  95                  100

GGA AAA AAG GGC GTT TTA TTC TGG CAA ATT ATT AGA TAT ATT CAA AAT        2367
Gly Lys Lys Gly Val Leu Phe Trp Gln Ile Ile Arg Tyr Ile Gln Asn
            105                 110                 115

ACA TTT CCT AAA TAC TTG TTG CTT GAA AAT GTT GAT AGA TTA TTG AAA        2415
Thr Phe Pro Lys Tyr Leu Leu Leu Glu Asn Val Asp Arg Leu Leu Lys
            120                 125                 130

TCA CCT TCG AGT CAG AGA GGG AGA GAC TTT GCT GTA ATG TTA TCA ACC        2463
Ser Pro Ser Ser Gln Arg Gly Arg Asp Phe Ala Val Met Leu Ser Thr
            135                 140                 145

TTA AAT GAG TTA GGC TAT AAT GTT GAA TGG CGC GTG ATT AAT GCT GCT        2511
Leu Asn Glu Leu Gly Tyr Asn Val Glu Trp Arg Val Ile Asn Ala Ala
        150                 155                 160

GAT TAT GGC AAT GCT CAA AGA CGT AGA AGG GTA TTT ATA TTT GGA TAT        2559
Asp Tyr Gly Asn Ala Gln Arg Arg Arg Arg Val Phe Ile Phe Gly Tyr
165                 170                 175                 180

AAG CAA GAT TTA AAC TAT AGC AAA GCT ATG GAA GAA AGT CCG TTG GAT        2607
Lys Gln Asp Leu Asn Tyr Ser Lys Ala Met Glu Glu Ser Pro Leu Asp
            185                 190                 195

AAA ATT ATA TAT CAC AAT GGT TTG TTT GCT GAA GCT TTT CCG ATT GAA        2655
Lys Ile Ile Tyr His Asn Gly Leu Phe Ala Glu Ala Phe Pro Ile Glu
            200                 205                 210

GAT TAT GCC AAT AAA AAT AGA GTA AAT AGG ACT CAT ATT ACT CAT GAT        2703
Asp Tyr Ala Asn Lys Asn Arg Val Asn Arg Thr His Ile Thr His Asp
            215                 220                 225

ATA GTC GAT ATT TCA GAT AAT TTC AGT TTT CAA TTT TAT AAT AGT GGA        2751
Ile Val Asp Ile Ser Asp Asn Phe Ser Phe Gln Phe Tyr Asn Ser Gly
        230                 235                 240

ATC ATG AAA AAT GGA GAA ATT TTA ACT ATT GAC ACA ATA CCA AAA TAT        2799
Ile Met Lys Asn Gly Glu Ile Leu Thr Ile Asp Thr Ile Pro Lys Tyr
245                 250                 255                 260

GAA AAA TCA GTA ACC TTA GGA GAA ATT ATT GAA AGT AAT GTA GAT GAT        2847
Glu Lys Ser Val Thr Leu Gly Glu Ile Ile Glu Ser Asn Val Asp Asp
            265                 270                 275
```

```
GGT TTT TCA TTA AAT CAA GAT CAA ATT GAT AAA TTT AAA TAT TTA AGA    2895
Gly Phe Ser Leu Asn Gln Asp Gln Ile Asp Lys Phe Lys Tyr Leu Arg
            280             285             290

GGA CCC AAA AAG ATT AAA CGA ACT ACT AAA GAT GGT CAT GAA TAT TAT    2943
Gly Pro Lys Lys Ile Lys Arg Thr Thr Lys Asp Gly His Glu Tyr Tyr
            295             300             305

TTT TCA GAA GGT GGT ATG TCT GAA ACA GAT TCA TTA GAG TTA CCT GCA    2991
Phe Ser Glu Gly Gly Met Ser Glu Thr Asp Ser Leu Glu Leu Pro Ala
            310             315             320

AGA ACA ATG CTT ACA AGT GAA TCA TCT ATT AAT AGA AGT ACT CAT TTT    3039
Arg Thr Met Leu Thr Ser Glu Ser Ser Ile Asn Arg Ser Thr His Phe
325             330             335             340

TTA AAC GTA GAT GGT GTT TAT AGA ACT TTG ACA CCT ATT GAA GCA GAA    3087
Leu Asn Val Asp Gly Val Tyr Arg Thr Leu Thr Pro Ile Glu Ala Glu
345             350             355             360

AGG TTG AAT GGG TTT CCA GAT AAT TGG ACA GAA GGT ATG CCA ATT AAG    3135
Arg Leu Asn Gly Phe Pro Asp Asn Trp Thr Glu Gly Met Pro Ile Lys
            365             370             375

ATG AGA TAC TTT TGT ATG GGC AAT GCT CTT GTT GTG CCT TTG ATT ACT    3183
Met Arg Tyr Phe Cys Met Gly Asn Ala Leu Val Val Pro Leu Ile Thr
            380             385             390

AGA ATA GGT AAT CAA ATT GAA AAA ATT GAT AGT ATT ACA AAT GAT GAA    3231
Arg Ile Gly Asn Gln Ile Glu Lys Ile Asp Ser Ile Thr Asn Asp Glu
            395             400             405

TTC AGT CAG CTA CGT TTA TTT TAAATAAATC AAATGTAGAG TGCGTTTGAT        3282
Phe Ser Gln Leu Arg Leu Phe
            410             415

TTAACAAGTT TGTATACTAA AGATTCATAG ATTATTGTAT ATTGACCAGT ATTTATCAGC   3342

GTATTATTTT AATATATA                                                 3360
```

## Patentansprüche

1. DNA,
   **dadurch gekennzeichnet,**
   daß sie
   (1) mindestens einen der beiden für ein Protein mit einer Sau3AI-Methylase-Aktivität bzw. für ein Protein mit einer Sau3AI-Endonuklease-Aktivität kodierenden Bereiche der Nukleotidsequenz SEQ ID NO:1,
   (2) eine der DNA aus (1) im Rahmen der Degeneration des genetischen Codes entsprechende Sequenz oder
   (3) eine Sequenz, die unter stringenten Hybridisierungsbedingungen mit einer DNA aus (1) oder (2) hybridisiert,
   enthält.

2. DNA nach Anspruch 1,
   **dadurch gekennzeichnet,**
   daß innerhalb oder/und außerhalb des für ein Protein mit einer Sau3AI-Methylase-Aktivität kodierenden Bereichs eine oder mehrere Mutationen eingeführt sind, welche eine Reduzierung der Gesamt-Methylase-Aktivität bei Expression in einer Zelle bewirken.

3. DNA nach Anspruch 2,

**dadurch gekennzeichnet,**

daß innerhalb des für ein Protein mit einer Sau3AI-Methylase-Aktivität kodierenden Bereichs, insbesondere innerhalb der ersten 1000 Basen ein Stopcodon eingeführt ist, hinter dem vorzugsweise mindestens ein A-Rest folgt.

4. DNA nach Anspruch 3,
**dadurch gekennzeichnet,**
daß das CGA-Codon an Position 2134-2136 der Nukleotidsequenz SEQ ID NO:1 durch ein TGA-Codon ersetzt ist.

5. Rekombinanter Vektor,
**dadurch gekennzeichnet,**
daß er eine oder mehrere Kopien einer DNA nach einem der Ansprüche 1 bis 4 enthält.

6. Rekombinanter Vektor nach Anspruch 5,
**dadurch gekennzeichnet,**
daß er ein Sau3AI-Endonuklease-Gen oder/und ein Sau3AI-Methylase-Gen unter Kontrolle eines regulierbaren dichten Promotors enthält.

7. Rekombinanter Vektor nach Anspruch 6,
**dadurch gekennzeichnet,**
daß der regulierbare dichte Promotor ein fdhF-Promotor, ein pdoc-Promotor oder ein T7-Promotor ist.

8. Rekombinanter Vektor nach einem der Ansprüche 5 bis 7,
**dadurch gekennzeichnet,**
daß er 5'-stromaufwärts einer DNA nach einem der Ansprüche 1 bis 4 einen Transkriptionsterminator enthält.

9. Mikroorganismus,
**dadurch gekennzeichnet,**
daß er mit einer DNA nach einem der Ansprüche 1 bis 4 oder mit einem oder mehreren rekombinanten Vektoren nach einem der Ansprüche 5 bis 8 transformiert ist.

10. Staphylococcus carnosus, transformiert mit dem Plasmid pSEM7 (DSM 5875).

11. Verfahren zur Gewinnung einer DNA nach Anspruch 1,
**dadurch gekennzeichnet,**
daß man
(1) eine Genbank aus chromosomalen DNA-Fragmenten eines Ausgangsorganismus, welcher das Sau3AI-Restriktions-Modifikationssystem besitzt, durch Transformation eines, mit dem Ausgangsorganismus nahe verwandten Organismus herstellt, welcher ursprünglich kein Sau3AI-Restriktions-Modifikationssystem besitzt,
(2) Klone des transformierten Organismus auf Sau3AI-Methylase-Aktivität testet und
(3) positive Klone mit Sau3AI-Methylase-Aktivität isoliert, welche zusätzlich ein Gen enthalten können, das für ein Protein mit einer Sau3AI-Endonuklease-Aktivität kodiert.

12. Verfahren nach Anspruch 11,
**dadurch gekennzeichnet,**
daß man als Ausgangsorganismus Staphylococcus aureus 3AI verwendet.

13. Verfahren zur Gewinnung eines rekombinanten Proteins mit Sau3AI-Endonuklease-Aktivität,
**dadurch gekennzeichnet,** .
daß man einen Mikroorganismus nach Anspruch 9 oder 10, der eine oder mehrere Kopien eines Sau3AI-Endonuklease-Gens enthält, in einem geeigneten Medium unter geeigneten Expressionsbedingungen kultiviert und das Protein aus dem Zellextrakt oder Medium anreichert.

14. Rekombinantes Protein mit Sau3AI-Endonuklease-Aktivität,
**dadurch gekennzeichnet,**

daß es durch das Verfahren nach Anspruch 13 aus einem transformierten Mikroorganismus erhältlich ist.

## Abb.1

pSEM7
14300bp

## Abb.2

pTSM1
8422 bp

## Abb.3

## Abb.4

Abb.5

# Abb.6

# Abb.7

Europäisches
Patentamt

**EUROPÄISCHER
RECHERCHENBERICHT**

Nummer der Anmeldung

**EP 91 10 9302**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | GENE Band 94, Nr. 1, 28. September 1990, Seiten 37-43, Amsterdam, NL; S. SEEBER et al.: Cloning, expression and characterization of the Sau3AI restriction and modification genes in Staphylococcus carnosus TM300" <br> * das ganze Dokument * <br> — — — | 1,5-14 | C 12 N 15/55 <br> C 12 N 15/54 <br> C 12 N 9/22 <br> C 12 N 9/10 |
| D,Y | NUCLEIC ACIDS RESEARCH Band 3, Nr. 11, November 1976, Seiten 3193-3202, London, GB; J.S. SUSSENBACH et al.: "A restriction endonuclease from Staphylococcus aureus" <br> * das ganze Dokument * <br> — — — | 1,5,9,11, 13,14 | |
| Y | EP-A-0 193 413   (NEW ENGLAND BIOLABS, INC.) <br> * das ganze Dokument * <br> — — — | 1,5,9,11, 13,14 | |
| A | JOURNAL OF BACTERIOLOGY Band 164, Nr. 2, November 1985, Seiten 501-509, US; R.M. BLUMENTHAL et al.: "Cloning of a Restriction-Modification System from Proteus vulgaris and Its Use in Analyzing a Methylase-Sensitive Phenotype in Escherichia coli" <br> * das ganze Dokument * <br> — — — | 1-5,9,11, 13,14 | |
| Y | BIOKHIMIYA Band 54, Nr. 6, Juni 1989, Seiten 1009-1014, Moskau, SU; A.J. LEBENKA et al.: "DNA Methylase Sau 3A Isolation and Properties" <br> * das ganze Dokument * <br> — — — | 1,5,9,11, 13,14 | |
| Y | EP-A-0 248 678   (NEW ENGLAND BIOLABS, INC.) <br> * das ganze Dokument * <br> — — — | 1,5,9,11, 13,14 | |
| Y | GENE Band 74, 1988, Seiten 25-32, Amsterdam, NL; K.D. LUNNEN et al.: "Cloning type-II restriction and modification genes" <br> * das ganze Dokument; insbesondere Seite 30, Spal te 2 - Seite 31, Spalte 1 * <br> — — — — — | 1,2,5,9, 11,13,14 | |

**RECHERCHIERTE SACHGEBIETE (Int. Cl.5)**

C 12 N 15/55
C 12 N 15/54
C 12 N 15/52
C 12 N 9/22
C 12 N 9/10

Der vorliegende Recherchenbericht wurde für alle Patentansprüche erstellt

| Recherchenort | Abschlußdatum der Recherche | Prüfer |
|---|---|---|
| Berlin | 29 Juli 91 | JULIA P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument